# EUROPÄISCHE PATENTSCHRIFT

(11) **EP 0 459 385 B1**
(45) Veröffentlichungstag und Bekanntmachung des Hinweises auf die Patenterteilung: **17.01.1996**
(21) Anmeldenummer: 91108669.2
(22) Anmeldetag: 28.05.1991
(51) Int. Cl.: C12N 15/56

(54) **Maltopentaose produzierende Amylasen**
Maltopentaose producing amylases
Amylases produisant de la maltopentaose

(30) Priorität: 31.05.1990 DE 4017595
(43) Veröffentlichungstag der Anmeldung: 04.12.1991
(73) Patentinhaber: Consortium für elektrochemische Industrie GmbH, D-81379 München (DE)
(72) Erfinder: Schmid, Gerhard, Dr., W-8000 München 60 (DE); Candussio, Anton, W-8000 München 50 (DE); Böck, August, Prof. Dr., W-8085 Kaltenberg (DE)

(56) Entgegenhaltungen:
- PATENT ABSTRACTS OF JAPAN, DATABASE JAPS/JPO vol.12,no.270[C515],27.07.88 & JP-A- 63 049082 [OJI KOONSUTAAC KK.] 01.03.88
- AGRICULTURAL AND BIOLOGICAL CHEMISTRY vol. 49, no. 12, 1985, TOKYO JP Seiten 3369 - 3376; N.Yoshigi et al.: "Purification and properties of an amylase from Bacillus cereus NY-14"
- APPLIED MICROBIOLOGY AND BIOTECHNOLOGY vol. 25, 1986, BERLIN DE Seiten 137 - 142; A. Candussio et al.
- EUROPEAN JOURNAL OF BIOCHEMISTRY vol. 191, 1990, BERLIN DE Seiten 177 - 185; A. Candussio et al.: "Biochemical and genetic analysis of a maltopentaose- producing amylase from an alkaliphilic Gram-positive bacterium"

## Beschreibung

Die Erfindung betrifft Maltopentaose (G5) produzierende Amylasen und Derivate davon.

Außer Glucose (Glucoamylasen) und Maltose (β-Amylasen) können nur sehr wenige Maltooligosaccharide in ausreichendem Reinheitsgrad direkt durch die Hydrolyse von Stärke mittels Amylasen gewonnen werden. α-Amylasen produzieren bei der Stärkehydrolyse in der Regel Gemische aus Glucose und niedermolekularen Maltooligosacchariden (G2-G9). Eine Reinigung einzelner Komponenten aus solchen Gemischen ist aufwendig und teuer. Einzelne α-Amylasen besitzen jedoch ausreichend hohe Produktspezifitäten um sie zur technischen Produktion definierter Oligosaccharide verwenden zu können. Bisher sind 3 G5 bildende Amylasen bekannt:

### a) Bacillus licheniformis

- US-Patent 4,039,383 vom 2. August 1977
- Arch. Biochem. Biophys., 155, 290-298, (1973)

Das Enzym aus dem thermophilen Organismus Bacillus licheniformis besitzt ein Temperaturoptimum von 70°C und ist in einem weiten pH-Bereich von pH 4,0-10,0 aktiv. Das Molekulargewicht (MW) beträgt 22,5 kDä. Als Produkte bei der Amylosehydrolyse entstehen zunächst längerkettige Maltooligosaccharide (G5-Gn), die im weiteren Verlauf der Reaktion jedoch weiter in das Hauptprodukt G5 und in größeren Mengen auch in G1-G4 abgebaut werden. Das US-Patent 4 039 383 vom 2. August 1977 beschreibt ein Verfahren um Amylose (ein schlecht wasserlösliches Substrat) zu hydrolysieren und löslich zu machen. Die gelöste Amylose wird dann als Substrat für die aufgereinigte Amylase zur G5-Produktion verwendet. Auf Grund der vielen Nebenprodukte muß das Produktgemisch nach der Enzymreaktion durch Chromatographie aufgereinigt werden.

### b) Bacillus cereus NY-14

- JP 158099 vom 13. September 1982 (entspricht US 4 591 561 vom 27. Mai 1986)
- JP 142330 vom 3. August 1983
- Agric. Biol. Chem., 49 (12), 3369-3376, (1985) (ABC)

In der angebenen Literaturstelle (ABC) wird die Reinigung und Charakterisierung einer 55 kDa Amylase aus Bacillus cereus NY-14 beschrieben, die ein pH-Optimum von pH 6,0 und ein Temperaturoptimum von 55°C besitzt. Das Enzym spaltet zunächst Stärke in die Maltooligosaccharide G3-G8. Die längerkettigen Zucker werden sekundär dann zu G1-G5 abgebaut. Das Patent JP 158099 vom 13. September 1982 (entspricht US-4591561 vom 27. Mai 1986) beschreibt die Produktion von G5 dadurch, daß ein Bacillus Stamm (hier NY-14) in einem Medium angezogen wird, das ein Substrat enthält (Stärke, Amylose usw.), welches von Enzymen des verwendeten Organismus in Maltooligosaccharide gespalten werden kann. Die Gewinnung definierter Oligosaccharide erfolgt bei diesem Verfahren durch Filtration der Kulturbrühe und anschließende Chromatographie.

Das Patent JP 142330 vom 3. August 1983 schützt das G5-spezifische Enzym aus Bacillus cereus NY-14. Bei der Beschreibung des Enzyms tritt ein Widerspruch zu der Beschreibung in ABC auf, da das MW des Enzyms im Patent mit 90 kDa angegeben wird, in der Veröffentlichung jedoch mit 55 kDa.

### c) Pseudomonas sp. KO 8940

- JP 044069 vom 9. März 1984
- JP 044070 vom 9. März 1984
- JP 253786-87 (Div ex 044069-84)
- Appl. Microbiol. Biotechnol., 25, 137-142, (1986)
- Agric. Biol. Chem., 54(1), 147-156, (1990)

In der Arbeit in Appl. Microbiol. Biotechnol. beschreiben die Autoren in erster Linie das Pseudomonas Isolat KO 8940 und die Bedingungen, unter denen es die G5-Amylase bildet. In der neuesten Publikation (Agric. Biol. Chem. 54(1), 147-156 (1990)) wird die Reinigung und biochemische Charakterisierung wahrscheinlich dieser G5-Amylase beschrieben. Die Amylase aus dem Pseudomas-Isolat KO 8940 wird jedoch nicht ausdrücklich genannt. Das gereinigte Enzym besitzt eine hohe initiale G5-Bildungsaktivität. Erst nach längeren Inkubationszeiten treten kürzere Hydrolyseprodukte auf.

Das Patent JP 253786-87 schützt das Enzym aus Pseudomonas KO 8940 und seinen Einsatz zur G5-Produktion. Die Amylase besitzt demnach ein Temperaturoptimum von 45°C bis 55°C und ein pH-Optimum bei pH 6,0-7,0. Ihr MW beträgt 72,5 kDa.

In Patent JP 044070 vom 9. März 1984 wird der Amylaseproduzent Pseudomonas KO 8940 geschützt.

Zur Gewinnung von Maltopentaose unter Verwendung der bekannten Enzyme werden entweder aufwendig gereinigte Enzyme verwendet, oder die Maltopentaose wird aufwendig aus dem Kultursubstrat aufgereinigt.

Gegenstand der Erfindung sind die Maltopentaose produzierende Amylase (A-180)gemäß Anspruch 1 sowie Verfahren zur Herstellung von bestimmten Derivaten dieser Amylase. Ein weiterer Gegenstand der Erfindung sind DNA Konstrukte kodierend bestimmte Derivate der Amylase gemäß Anspruch 1. Ein weiterer Gegenstand der Erfindung ist das Isolat 163-26 (DSM 5853; DSM GmbH, 3300 Braunschweig, Mascheroder Weg 1b).

Innerhalb der erfindungsgemäßen Verfahren können Bakterien, bevorzugt alkalophile stärkeabbauende Bakterien, in bekannter Weise auf ihre Fähigkeit zur Produktion von Maltopentaose aus Stärke gescreent werden.

Bakterien mit dieser Eigenschaft werden charakterisiert. So wurde das Isolat 163-26 erhalten. Das amylolytische Enzym aus diesem Isolat wurde aufgereinigt und biochemisch charakterisiert.

Um größere Mengen des Enzyms in Prokaryonten, bevorzugt E. coli, herstellen zu können, wird das das Enzym kodierende Gen in einen Vektor, bevorzugt ein Plasmid, kloniert, und in bekannter Weise sequenziert. Das kodierende Gen wird durch gezielte Mutagenese so modifiziert, daß es in geeigneten Prokaryonten die Exkretion des als Amylase funktionstüchtigen modifizierten Proteins in großen Mengen ermöglicht.

Um dies zu erreichen, wird das Strukturgen im Plasmid unter die Kontrolle eines induzierbaren Promotors, bevorzugt unter die Kontrolle des Lactose induzierbaren tac-Promotors, gestellt. Damit wird eine starke, steuerbare Überproduktion der Amylase ermöglicht. Um zu verhindern, daß das Enzym intrazellulär abgebaut wird, und um die Verwendung des Enzyms ohne aufwendige Isolierungsverfahren zu ermöglichen ist die effektive Sekretion des Enzyms in das Kulturmedium wünschenswert.

Um dies zu erreichen, fusioniert man unter Erhaltung des Leserahmens die kodierende Region für das Signalpeptid eines sekretierbaren Enzyms, bevorzugt das Signalpeptid der CGTase aus Klebsiella oxytoca, mit dem Strukturgen des Enzyms. Beispielsweise durch den Vergleich der Proteinsequenz mit den Sequenzen bekannter Amylasen lassen sich funktionell wichtige Enzymdomänen und für die Funktion unwesentliche Proteinbereiche abschätzen und daraus folgernd weitere Modifikationen des Strukturgens durchführen, die unter Beibehaltung der Produktspezifität zu einer verstärkten Enzymexkretion in das Kulturmedium führen oder eine erhöhte Enzymstabilität im Kulturmedium bewirken.

Somit ist eine Aufreinigung oder Konzentration des Enzyms aus dem Kulturüberstand unnötig. Der Kulturüberstand kann direkt zur Maltopentaose-Produktion verwendet werden. Durch geeignete Wahl der Reaktionsbedingungen lassen sich dann Verfahren etablieren, bei denen die Maltopentaoseausbeute so hoch ist, daß eine Aufreinigung der Maltopentaose unnötig ist. Bei G5-Ausbeuten von über 90% kann eine weitere Aufreinigung der Maltopentaose unterbleiben. Eine einfache Gewinnung der Maltopentaose aus den Hydrolyseansätzen z.B. durch Sprühtrocknung ist möglich.

In den Beispielen wird die Isolierung einer erfindungsgemäßen Maltopentaose produzierenden Amylase, ihre DNA-Sequenz, ihre gentechnologische Modifizierung sowie ihre Expression in E. coli beschrieben. Weiter werden Beispiele der Stärkekonversion unter Verwendung der Amylase und ihrer gentechnisch veränderten Modifikationen gegeben.

- Fig. 1:: Schematische Darstellung des Plasmids pACA1 Ein 7,9 kb Fragment chromosomaler DNS aus Isolat 163-26 wurde in die BamHI/XmaIII-sites des Plasmids pAC1 (pACYC184-Derivat) kloniert. Das in dem Fragment enthaltene A-180 - Strukturgen wurde zur Konstruktion der im Text beschriebenen Mutanten verwendet.
- Fig. 2:: Restriktionskarte des DNA - Fragmentes, das aus Isolat 163-26 in den Vektor pAC1 kloniert wurde (pACA1).
Die schematische Darstellung zeigt die Mutationen, die zu den Expressionsplasmiden pEX1051 und pEX21 führten.

### Beispiel 1: Screening nach Maltopentaose produzierenden alkalophilen Bakterien

Bodenproben aus verschiedensten Regionen der Erde wurden gesammelt. 0,1-0,2 g Erde wurden abgewogen und in sterilen Gefäßen mit 1 ml steriler physiologischer Kochsalzlösung aufgeschlämmt. Nach Absitzen der Grobanteile wurden jeweils 0,1 ml auf eine Stärkeagarplatte (10 g/l lösliche Stärke; 5 g/l Pepton; 5 g/l Hefeextrakt; 1 g/l KH₂PO₄; 0,2 g/l MgSO₄ x 7H₂O; 10 g/l Na₂CO₃; 15 g/l Agar; pH 10,4) plattiert. Die Inkubation der Agarplatten erfolgte bei 30°C für 2-3 Tage. Kolonien von stärkeabbauenden Bakterien zeigten einen trüben Halo, der durch Retrogradierung niedermolekularer Stärkemoleküle entstand. Die Kolonien wurden isoliert und zweimal auf Stärkeagarplatten gereinigt. Daraufhin wurde eine Anzucht in 2 ml Flüssigmedium obiger Zusammensetzung durchgeführt. Nach 48 h Inkubation bei 30°C wurden die Zellen abzentrifugiert und der Überstand auf Amylase-Aktivität getestet. 200 µl Überstand wurden mit 200 µl 10%-iger Stärkelösung in 20 mM Tris/Cl pH 9,0; 5 mM CaCl₂ 1 - 5 h bei 40°C inkubiert. Der Enzymtest wurde durch Zugabe von 600µl Methanol gestoppt und der Überstand nach Zentrifugation mittels HPLC analysiert. Aus einer Vielzahl von Isolaten zeigte nur der Stamm 163-26 die gesuchte Enzymaktivität.

### Beispiel 2: Stammcharakterisierung

Folgende Merkmale charakterisieren das alkalophile Isolat 163-26.

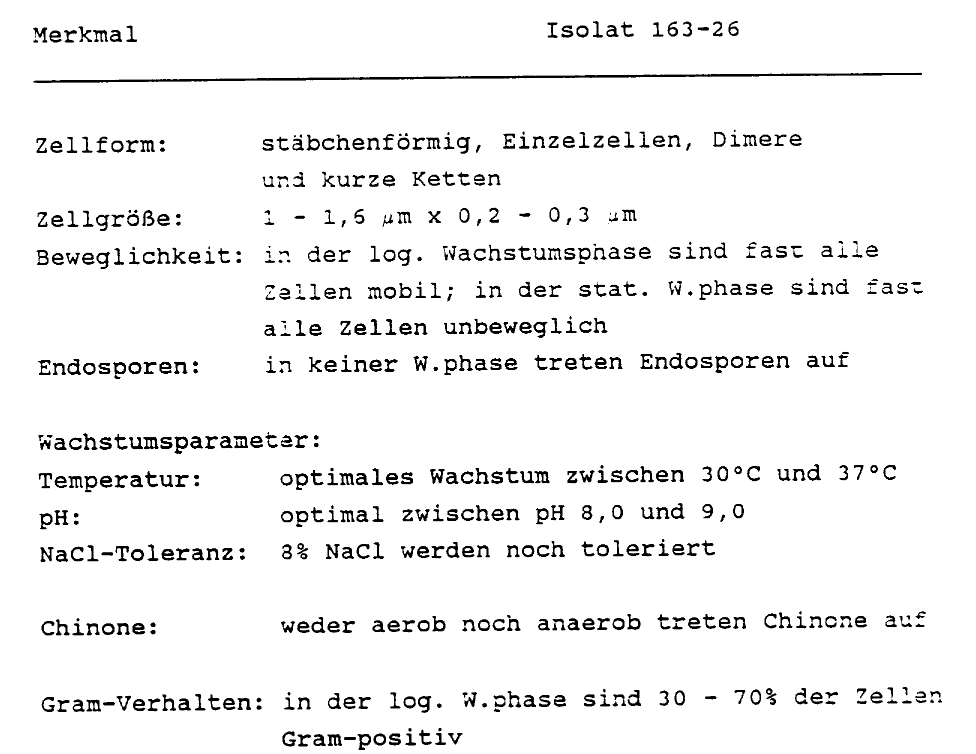

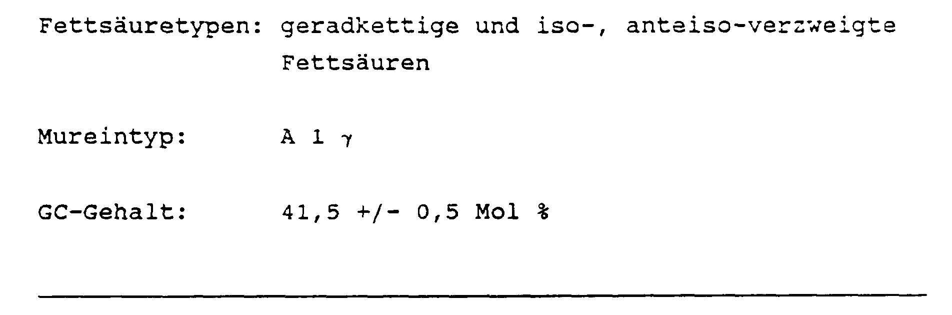

### Beispiel 3: Reinigung und Charakterisierung der Amylase A-180

### Beispiel einer typischen Reinigungsprozedur:

Isolat 163-26 wurde 20 Stunden in 40 l Medium M3/1 (5 g/l Noredux 150B; 5 g/l Pepton aus Casein; 5 g/l Hefeextrakt; 5 g/l NaCl; 3,5 g/l Na₂CO₃; 1 g/l KH₂PO₄; 0,2 g/l MgSO₄) aerob bei 37°C angezogen. Nach 20 Stunden wurde die Kultur durch die Zugabe von Eis rasch auf 4°C abgekühlt. Die Zellen wurden aus der Kulturbrühe durch Querstrom-Mikrofiltraticn an einer Millipore-Filterkassette (Porengröße 0,2µm) entfernt. Die Proteine im zellfreien Kulturüberstand wurden durch Ultrafiltration über eine Filtron Filterkassette (Trenngrenze 10 kDa) auf ein Volumen von 1 1 konzentriert. Die Lösung wurde anschließend durch Zugabe von gepulvertem Ammoniumsulfat auf eine 60%ige Ammoniumsulfatsättigung gebracht. Die dabei ausgefallenen Proteine wurden abzentrifugiert, in 50 ml TC-Puffer (20 mM Tris/Cl pH 7,2; 5 mM CaCl₂) gelöst und gegen TC-Puffer dialysiert. Die amylolytischen Enzyme in dieser Lösung wurden durch Stärkeadsorption gereinigt. Dazu wurde die Proteinlösung nach der Dialyse auf eine 20%-ige Ammoniumsulfatsättigung gebracht und mit 3 % löslicher Stärke versetzt. Der Ansatz wurde 3 Stunden bei 4°C gerührt und anschließend zentrifugiert. Der Niederschlag wurde in 1/2 Ausgangsvolumen Waschpuffer (20%ige Ammoniumsulfatsättigung, 1 M NaCl in TC-Puffer) suspendiert, 10 min bei 4°C gerührt und erneut zentrifugiert. Der hier erhaltene Niederschlag wird in 1 Ausgangsvolumen Elutionspuffer (3 M NaCl; 0,1 M Maltose in TC-Puffer) suspendiert und 2 h bei 4°C gerührt. Anschließend wird die Stärke abzentrifugiert und der Überstand gegen TC-Puffer dialysiert. Nach der Dialyse werden die Proteine in der Lösung durch Zugabe von Ammoniumsulfat (60%-ige Sättigung) gefällt, in TC-Puffer gelöst und erneut dialysiert. Die nun vorliegende Lösung enthält nur noch die von dem Isolat 163-26 gebildete α-Amylase A-60 und die Maltopentaose produzierende Amylase A-180. Die beiden Enzyme können durch Gelfiltrationen an einer Molekülsiebsäule TSK SW3000G (LKB) voneinander getrennt werden.

### Charakterisierung der Amylase A-180

Die MW-Bestimmung durch SDS-Polyacrylamidgelelektrophorese (PAGE) ergab ein MW von ca. 180 kDa für die Amylase A-180. Durch isoelektrische Fokussierung wurde ein isoelektrischer Punkt von 4,65 für das gereinigte Enzym ermittelt.
Eine Kinetik der Produktbildung bei der Stärkehydrolyse ergab eine initial sehr hohe G5-Spezifität für die Amylase A-180. A-180 besitzt ein biphasisches pH-Optimum bei pH-Werten von 6,0 und 8,5. Eine irreversible Inaktivierung von A-180 erfolgt erst bei pH Werten unter 5,5 oder über 11,0. Die optimale Temperatur für die Stärkehydrolyse liegt bei 55°C, allerdings ist das Enzym bei dieser Temperatur bereits leicht instabil, so daß zur G5-Produktion eine Temperatur von 45°C verwendet wird.
γ-Cyclodextrin kann von Amylase A-180 nicht hydrolysiert werden. Dieses Ergebnis zusammen mit dem Befund der hohen G5-Spezifität zeigt, daß es sich bei A-180 um eine Exo-maltopentaohydrolase handelt.

### Beispiel 4: Klonierung und Sequenzierung des A-180 - Strukturgens

### Klonierung

Um eine A-180 spezifische Sonde zu erhalten, mit deren Hilfe das Strukturgen identifiziert werden kann, wurde zunächst die N-terminale Aminosäuresequenz der gereinigten Amylase A-180 durch automatisierten Edman-Abbau (Gasphasen-Sequenator) bestimmt. Die durch die Sequenzierung erhaltene Aminosäuresequenz lautet:
Gln Glu Tyr Arg Glu Leu Asn Gln Leu Glu Asn Lys Pro Phe Ser Trp Asp Asn Ala Asn Val Tyr Phe Val Leu.

Durch reverse Translation konnte aus einem Teil dieser sequenz (Trp Asp Asn Ala Asn Val) eine 17 Basen lange Nukleotidsequenz abgeleitet werden, die im A-180 - Strukturgen enthalten sein muß. Die genaue Sequenz dieses Oligonukleotidgemisch lautet: 
5'- TGG GAC AAC GCA AAC GT - 3'
          T   T   T   T
                  C
                  G
 Diese Oligonukleotidsequenz (ein 32-fach degeneriertes 17-mer) wurde mit Hilfe eines DNS-Synthesizers hergestellt und mit ³P-γ-ATP radioaktiv markiert.

Chromosomale DNS von Isolat 163-26 wurde mit verschiedenen Restriktionsenzymen geschnitten, durch Elektrophorese in einem 0,8% Agarosegel aufgetrennt und auf eine Nylonmembran übertragen (Southern blot). Mit Hilfe des radioaktiven Oligonukleotidgemisches konnte durch Hybridisierungsstudien ein 2,7 kB ClaI-Fragment markiert werden, das für den N-terminalen Bereich von A-180 kodiert. Das ClaI-Fragment wurde isoliert, in den mit ClaI geschnittenen Vektor pBR322 ligiert und in E. coli HB 101 transformiert. Klone, die das richtige Insert enthielten wurden durch Hybridisierung ihrer Plasmid-DNS mit dem radioaktiven Oligonukleotidgemisch identifiziert. Mit Hilfe des klonierten DNS-Fragmentes, das nun markiert und als Hybridisierungsprobe verwendet wurde, konnte das gesamte A-180 - Strukturgen kloniert werden.

### Sequenzierung

Zur Bestimmung der Nukleotidsequenz des A-180 - Strukturgens wurden überlappende Fragmente des Gens in das Plasmid pUC19 subkloniert. Die Sequenz der Subklone wurde mit Hilfe von universellen oder internen Sequenzprimern nach der dideoxy-Kettenabbruchmethode bestimmt. Ein Ausdruck der kompletten A-180 Nukleotidsequenz und der abgeleitete Aminosäuresequenz zusammen mit den 5' und 3' flankierenden Bereichen des Gens ist im Folgenden dargestellt.

1 GGTACCGCCTATCTCAGTGTGTGAAAGCTATGCATCAAAATACCTACTCCATGAGCGTTT

61 CTTCAACACGAATCTACTTTATTTAATATTATTCATAACGAAACATCAGAAAATATTGTT

121 ATTACCTAAATTCCTTGTTTTTGTCTTTTAATGTTGGTCAATGTTCTATGGTTGTGCTAA

181 TAAAAATGTTAACGCTTTCTCAGGAGGCTATATGAGAGGGGTGATGTCTGCTAAACAATA

241 AGGATTCATCAACACCATGGTTATAAAAAATTAAAGATTGAAAGGAGGAAAAGGTAATGA
Shine-Dalgarno M

.

301 AGCAACAGCTTAATCGCGTGATAAGTATCGTATTATGTTTAATTGTCATGCTCTCGGTGT
K Q Q L N R V I S I V L C L I V M L S V

361 TTGAAAGTACTATTATGTTATTACCAGGTTCAGTAGAGGTAAAAGGCCAAGAGTATCGAG
F E S T I M L L P G S V E V K G Q E Y R

421 AACTAAATCAGCTAGAAAATAAACCTTTTTCATGGGATAATGCAAACGTTTACTTTGTGT
E L N Q L E N K P F S W D N A N V Y F V

481 TAACCGATCGTTTTTACAATGGAAATACAAGTAATGATAATTCTTATGGAGACCGCAAA
L T D R F Y N G N T S N D N S Y G R P Q

541 TAGATGCTTGGGGTACAAACATTGGTACTTTCCATGGCGGGGACATAAAAGGATTAACAA
I D A W G T N I G T F H G G D I K G L T

601 AGAAATTGGAAGAAGGTTACTTTACAGACCTAGGTACAAATGCCATATGGATATCTGCTC
K K L E E G Y F T D L G T N A I W I S A

661 CATGGGAACAAATGCATGGCTGGGTTGGTGGAAAGATGGTGATTTTGCTCACTATGGCT
P W E Q M H G W V G G K D G D F A H Y G

721 ATCATGGTTACTATGGATTAGATTTTACGGCTATGGATCAGAATATGGGTACAATTGATG
Y H G Y Y G L D F T A M D Q N M G T I D

781 AAATGCGTGAATTTGTTGACCTTGCACATTCATTAGGCATTAGAGTTGTTCTCGACATTG
E M R E F V D L A H S L G I R V V L D I

841 TTATGAATCACGTTGGCTATCCAACGATCGTTGACATGCATGAATTTGGTTTTGGTGATA
V M N H V G Y P T I V D M H E F G F G D

901 CTGGAGGACTTCCAAGAGATTGGACACCTAATCAAGCACAGGGCCAAAATTGGCATACAC
T G G L P R D W T P N Q A Q G Q N W H T

961 ATAATGACATTATGAATAAAGACAATGAAGCAGCTTGGGCGAATTGGTGGGGAAGTGACT
H N D I M N K D N E A A W A N W W G S D

1021 GGATTCGTGCCGATGAAACAGCCGGGTATGACAATTGTGGTGGCAGCGAACAGACAATGT
W I R A D E T A G Y D N C G G S E Q T M

1081 GTATAGGGTTCTTACCAGATATTAAAACGGAAGTAACCACAGGTGTTGATTTACCACCGA
C I G F L P D I K T E V T T G V D L P P

1141 TATTGAGAAACAAGTGGAATGATCAAGCTAGTGGCTATGAAGATTGGTTTGTTCCAGCAG
I L R N K W N D Q A S G Y E D W F V P A

1201 CTGAACCTTATCGTCAAGATTTAAACATTGCTCCGAAAGATTATTTGATCAAATGGATTA
A E P Y R Q D L N I A P K D Y L I K W I

1261 CTTCATGGGTTGAGGAATTCGGTATTGATGGATTCCGTGTTGATACAGCAAAGCATGTAG
T S W V E E F G I D G F R V D T A K H V

1321 AGATTGAGCGATGGGCTGAATTGAAGAATGAAGCGGAAGTAGCACTTCAAACATGGCGAG
E I E R W A E L K N E A E V A L Q T W R

1381 AAAATAACCCAGATAAGCCCGGTGCTAATTGGGATGATAATTTCTGGATGACAGCAGAAG
E N N P D K P G A N W D D N F W M T A E

1441 TATTTGGACATGGTCTTGGAAAAGCGAGTATTTTGATTTTGGTTTCGATTCTGTGATTA
V F G H G L G K S E Y F D F G F D S V I

1501 ATTTTGAATTCCAGAATGCAAACTTCAATAATTTAGAAGGTTTATTTTCTAGATATGCAA
N F E F Q N A N F N N L E G L F S R Y A

1561 ATTCAATTAACACTGACCCTGATTTCAACATGTTAAGTTATGTTTCATCTCATGATACAA
N S I N T D P D F N M L S Y V S S H D T

1621 AGCTTTATAGTAGAGATGACCTTATTCAGGCAGGTACAGCATTACTATTACTTCCAGGTG
K L Y S R D D L I Q A G T A L L L L P G

1681 GCGTTCAAGTGTTTTATGGCGATGAAACAGCTCGACCATTAGGGGATGGTGGTTCTGATC
G V Q V F Y G D E T A R P L G D G G S D

1741 CTGAGCAAGGTACGCGTTCATCGATGAATTGGGCTAATATTAATCAGAATGTACTCTCTC
P E Q G T R S S M N W A N I N Q N V L S

1801 ATTGGCAAAAACTTGGTCAATTCAGAAATAATCACATAGCTATTGGTGCGGGAGCGCATC
H W Q K L G Q F R N N H I A I G A G A H

1861 AGAAGTTATCTGATAGTCCGTATACGTTTGCGCGTACGTATGAATCAGACGATATAGTTG
Q K L S D S P Y T F A R T Y E S D D I V

1921 ATGAAGTCGTCGTTGCAACTGGGGCCCAAGGAACAACAGCTGTTACTGTAGAAGGTGTTT
D E V V V A T G A Q G T T A V T V E G V

1981 TTGAAGATGGGACAGTTGTTCGAGATGCTTATACTGGTGATGAGACAACAGTAACTAAAG
F E D G T V V R D A Y T G D E T T V T K

2041 GGACAGCAACATTTACTGCTGGAACACAAGGTATTATTCTAATCGAAAATACAGCTGAGC
G T A T F T A G T Q G I I L I E N T A E

2101 CAGTTACTAATTTGCCGATCGTTTCAGCAACACCTGGTAATAGTTCTTTTAGGACAGATG
P V T N L P I V S A T P G N S S F R T D

2161 ACATAACAATCACGCTAAATGTTGATCGAGCGGATATGGGGAAGTATACACTTGATGGAA
D I T I T L N V D R A D M G K Y T L D G

2221 GTGATCCAGCAGATGGCCTAACGTTTATGGATGGAGAAGAAATTGTCATTGGTGCTGATA
S D P A D G L T F M D G E E I V I G A D

2281 TGGAGTTTGATGAAACAGCAACATTGAGACTCTATGCAGAAAATGAAAATGGCATAAGAA
M E F D E T A T L R L Y A E N E N G I R

2341 CAAGGAGTTACACATATAGGAAGGTAGATCCAGATGCGTTACTTGAAGTATATTTTAAGA
T R S Y T Y R K V D P D A L L E V Y F K

2401 AACCAGCGGATTGGGGAACACCACATATATATTACTATGATACATTTCCAGAGGAGCCGG
K P A D W G T P H I Y Y Y D T F P E E P

2461 AAGTCACTTGGACTACAGCTCCAGAGATGACATTAGTAGAGGATGATTGGTATGTATATG
E V T W T T A P E M T L V E D D W Y V Y

2521 TTTTTGAAAATGCTGAAAGTGCCAATATAATATTTAAGGATTCTTCAGGAAAACAAATTC
V F E N A E S A N I I F K D S S G K Q I

2581 CAGGTCCAAATGAACCAGGATTCTTCATTGATCAGATTGGTTGGTACGATGGCGTAAAGT

P G P N E P G F F I D Q I G W Y D G V K

2641 GGCTTGATTCAGATCCTTTTGAAAGGGAACCTAAAGAGCCTGCGACAACACCTAAGAACC
W L D S D P F E R E P K E P A T T P K N

2701 TAAGTGTTGTTAATGTAACTGAAACTACTGTAACATTTGAGTGGGACCAATCTGATGGTT
L S V V N V T E T T V T F E W D Q S D G

2761 ATGTCGTTGAATACGAGATTTTACGTGATGAGGATGTTGTTGCTTCAACTATTCGTACAA
Y V V E Y E I L R D E D V V A S T I R T

2321 CATTTACGGATGAAGACCTTAATCCAGATACAACCTACACTTATTCTGTCGTAGCTGTTG
T F T D E D L N P D T T Y T Y S V V A V

2381 GAGAAGGCGGGCAGAAATCCGCCCCAAGTGAAGCGTTAAAAGTGACTACATTAGAAGAAA
G E G G Q K S A P S E A L K V T T L E E

2941 ATGATGAACCTAAGGAACCGGCTGAGGCGCCAGAAAATTTACGTATAGCTGATATAACAG
N D E P K E P A E A P E N L R I A D I T

3001 ATACAACAGTTACAATCAACTGGAATGCATCTAATGGTTACGTAACAGGATATGAGGTTC
D T T V T I N W N A S N G Y V T G Y E V

3061 TGCGTGATGGTGTGGTTATTGGCGAAACAACACGGACAACATTCATAGATACTGGATTAG
L R D G V V I G E T T R T T F I D T G L

3121 ATGCTGATAGGACCTATACGTATACGATTGTTGCTCTCGGAGATGGCGGGCAAAAGTCTG
D A D R T Y T Y T I V A L G D G G Q K S

3131 ATCCGAGCGAAGCGTTAGAAGTAACAACTCAAGAAAAACCAGAAGGAAATCTAGTAACAA
D P S E A L E V T T Q E K P E G N L V T

3241 TATACTATAAAAAAGGCTTTGATACCCCATATATGCATTATCGTCCGGAAGGTGGAGAGT
I Y Y K K G F D T P Y M H Y R P E G G E

3301 GGACGATCGTTCCAGGAATTAGAATGGAAGAATCAGAAATAGCAGGCTATAGTAAGTTAA
W T I V P G I R M E E S E I A G Y S K L

3361 CCGTTGATATTCGGGAAGCAAGCAAGTTGGAAGTAGCCTTTAATAATGGACGTGGGGCTT
T V D I R E A S K L E V A F N N G R G A

3421 GGGATAGTGATCAAGAGAATAATTATTTATTTGAGCCAGGTGTTCATACGTACATTCCGA
W D S D Q E N N Y L F E P G V H T Y I P

3481 GTCATGAAGGAAGAGGAGAGATTATTCCAGGTAAACCAGGAGCACCAATCGATGGTAATA
S H E G R G E I I P G K P G A P I D G N

3541 AAGTGACGATTTACTATCAAAATGGCTTTGATACGCCGTATGTTCATTACCGCCCAGAAG
K V T I Y Y Q N G F D T P Y V H Y R P E

3601 GCGGAAATTGGACCAACGCCCCAGGATTAAAAATGGAAGATTCAGAGTTTGCAAGTTATA
G G N W T N A P G L K M E D S E F A S Y

3661 GTAGGTTAACGCTTGATATTGGTGAAGCTAATCGTGCAGAAGTGGCTTTCAATAACGGAC
S R L T L D I G E A N R A E V A F N N G

3721 GCGGCCTTTGGGATAGTGATAATGAAAATAATTATTTCTTCAATATTGGCGATAACACTT
R G L W D S D N E N N Y F F N I G D N T

3781 ATATACCAGGAAAAAACGGTTCAGCTGGAGAGATTTATGGAGGTAAGCCAAGACCACCAT
Y I P G K N G S A G E I Y G G K P R P P

3841 TAGTAGGAAATGAAGTAATCATTTATTATAAAAATGGTTTTGATACACCGTATGTTCATT
L V G N E V I I Y Y K N G F D T P Y V H

3901 ATCGTCCAGAAGGTGGTACGTGGACAAATGCACCAGGAATAAAAATGGATAAGTCAGAAA
Y R P E G G T W T N A P G I K M D K S E

3961 TAGCAGGTTACAGTAAAATAACGCTTGATATTGGTCGCGCAGATCGAGTAGAAGTAGCCT
I A G Y S K I T L D I G R A D R V E V A

4021 TTAATGACGGTCGTGGTGCATGGGATAGTGATAACGAACGTAATTATCTCTTTGTAGTCG
F N D G R G A W D S D N E R N Y L F V V

4081 GTAACAATACTTATGAACCAGGAATTAACGGCGCACCTGGTCAGGTGAAACATGGCGTGT
     G  N  N  T  Y  E  P  G  I  N  G  A  P  G  Q  V  K  H  G  V

4141 TACCTGATGATGGAGAAGATCCGGGAGATATTGAAGACCCTGATCATACCTCCCCTTCAA
     L  P  D  D  G  E  D  P  G  D  I  E  D  P  D  H  T  S  P  S

4201 AGCCGACTGATTTAACAGCAATAGCTACTGCTCATACTGTTTCATTAAGCTGGACAGCTT
     K  P  T  D  L  T  A  I  A  T  A  H  T  V  S  L  S  W  T  A

4261 CAGCAGACGATGTAGAAGTAGCTGGGTACAAAATTTATCGAGATGGTGTGGAAATCGGTG
     S  A  D  D  V  E  V  A  G  Y  K  I  Y  R  D  G  V  E  I  G

4321 TTACTGAATCAACAACTTATACGGATTCAGGCTTAACGGCAGAAACAACGTATAGCTATA
     V  T  E  S  T  T  Y  T  D  S  G  L  T  A  E  T  T  Y  S  Y

4381 TGGTACAAGCTTATGATACTTCTAATAATTTCTCGGCATTAAGTGATGAACTGACAATTG
     M  V  Q  A  Y  D  T  S  N  N  F  S  A  L  S  D  E  L  T  I

4441 AAACCGCCGAGAAAACGGGTGTTGATCCAGGAGGGGATATGCCTTATTCCACGAATCCAT
     E  T  A  E  K  T  G  V  D  P  G  G  D  M  P  Y  S  T  N  P

4501 CGTTTGGTAAGAAGGTAACAACGCCAATCACAATTGATGGTGTTAATGACGGGGAATGGA
     S  F  G  K  K  V  T  T  P  I  T  I  D  G  V  N  D  G  E  W

4561 CAGATGATATGTTGATTGCAATTGGTATGGCTGGTGACGACCCACGTTCGCTCGGGGACA
     T  D  D  M  L  I  A  I  G  M  A  G  D  D  P  R  S  L  G  D

4621 ATTGGTCTATGCATGAAACACCAATGGACCTTACTCACCTATGGGGAGCATGGGACCATG
     N  W  S  M  H  E  T  P  M  D  L  T  H  L  W  G  A  W  D  H

4681 AGTACTTGTATCTTGCTTGGCAATATGTAGATGTAACAGATATTATTGACCCAGCTAACG
     E  Y  L  Y  L  A  W  Q  Y  V  D  V  T  D  I  I  D  P  A  N

4741 CAGGCTCATCAGCTGGTACCACAATTAGCCAGATGGATATGCCACAAACCATTGCAATTG
     A  G  S  S  A  G  T  T  I  S  Q  M  D  M  P  Q  T  I  A  I

4801 ATACCATCCCAGAGCAAGGTGCAACACATGATATGTGGGGAAAAATGGTGGTGAATCAC

D T I P E Q G A T H D M W G K N G G E S

4861 TTTGGGGAGGACCAGATTTACCTGATTACCAACTAAATATCGCATCTAATATGTTCCATT
L W G G P D L P D Y Q L N I A S N M F H

4921 CAGGCTATATTTCTAGAGCAGTTGATGGTGTATTTCCTGTTGACGATGGAGGAATAAATT
S G Y I S R A V D G V F P V D D G G I N

4981 ATAAAACGGGTGAGGAAGCAGGAATTACAGTAAAGTTTTCTAAAGGTAAAGGGTATTCAA
Y K T G E E A G I T V K F S K G K G Y S

5041 CATTGTGGGGGGTGTTAGATGCTGATGATGCAGTTGATCCTAGTAAACTTGTGAACTTCA
T L W G V L D A D D A V D P S K L V N F

5101 CCGAGCTTGCCCATGATTCAACACGAGATACTTTTTATGAAGCAAAGATTCCTTTAGCTG
T E L A H D S T R D T F Y E A K I P L A

5161 CAATTGGTAATCCCGACATTGAAAATGAACGCATTGGTGTCATGATTCATCAAGGTGAAT
A I G N P D I E N E R I G V M I H Q G E

5221 TTTCGCCGATGGACACGCTACCGAATGACCCCGCAACATCCGATACACCAGGTGTGAGTG
F S P M D T L P N D P A T S D T P G V S

5281 AATCAAATTCGCCATTAGAATGGGAAGACATTGACCTGTTAACAGTGCCATTTGCAAGAA
E S N S P L E W E D I D L L T V P F A R

5341 TTGGCCAATAATTATGAAATAAGCCGGCATGAGTCTATGCTGGCTTTTTGTACGGCTGGC
I G Q -          ==========>     <==========

5401 AGTTGCACATGCAGAGACGACACTGTGGTGTAACAGCTAGATGACAGTAAATCATTGGCA

5461 TTCCAGATGGTCGGAATAAAAGACGGATTTGTGTATATAGTAACTCTATTGATGAAGTTT

5521 TCCCGTTAGTTCCATCCTCAGATTATCAATCATCAATAATAGGTGGTTTCATGGATTGCT

5531 TAGGAAATATCGAGGATGACCTATTTACTGTTCACTAAATCTGATTAGAGTTTATTGGTA

5641 TCAAAAAAGCGTTATTTCTTCAAAGCAAGAAGCAGGCAACTGGGATTATCTACATTTCCC

5701 AATGATATGAAAAATGCTGTTAAAAAAGATGGTTGAATATT

Der offene Leserahmen, der für A-180 kodiert umfaßt 5052 Nukleotide, entsprechend 1684 Aminosäuren. Das abgeleitete MW von 186,5 kDa korrespondiert mit den 180 kDa die durch SDS-PAGE ermittelt wurden.

### Beispiel 5: Mutagenese des A-180-Strukturgens

Drei Mutationen waren notwendig, um das klonierte A-130-Strukturgen so zu verändern, daß eine massive Produktion, gekoppelt mit Export und proteolytischer Stabilität der G5-spezifischen Amylase in geeigneten E. coli-Stämmen erfolgt.

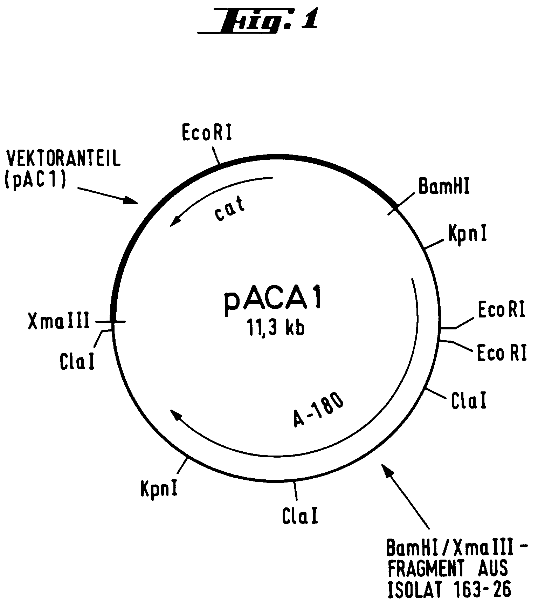

- Um eine massive Expression des A-180 - Strukturgens und damit eine starke Amylaseproduktion zu erhalten, die zusätzlich durch einfache Methoden steuerbar, d.h. induzierbar oder repremierbar ist, wurde das A-180 - Strukturgen unter Kontrolle eines neuen Promotors gesetzt.
Dazu wurde das A-180 - Strukturgen aus dem Plasmid pACA1 (Fig. 1) isoliert und hinter den 'tac'-Promotor in den Polylinker des Expressionsplasmids pJF118u (Gene 48, 119-131 (1986); Derivat von pkk 223, dieser ist erhältlich bei der Fa. Pharmacia, Freiburg) kloniert. Dieser Promotor wird durch das lacI^{q}-Gengroäukt (das ebenfalls auf pJF118u kodiert ist) solange repremiert, bis Induktoren wie Lactose oder analoge Verbindungen, z.B. IPTG, dem Medium zugesetzt werden.
Durch diese Mutation war zwar eine massive Produktion von A-180 möglich, allerdings war das rekombinante Genprodukt zu 100% im Cytoplasma von E. coli lokalisiert und wurde dort stark abgebaut.

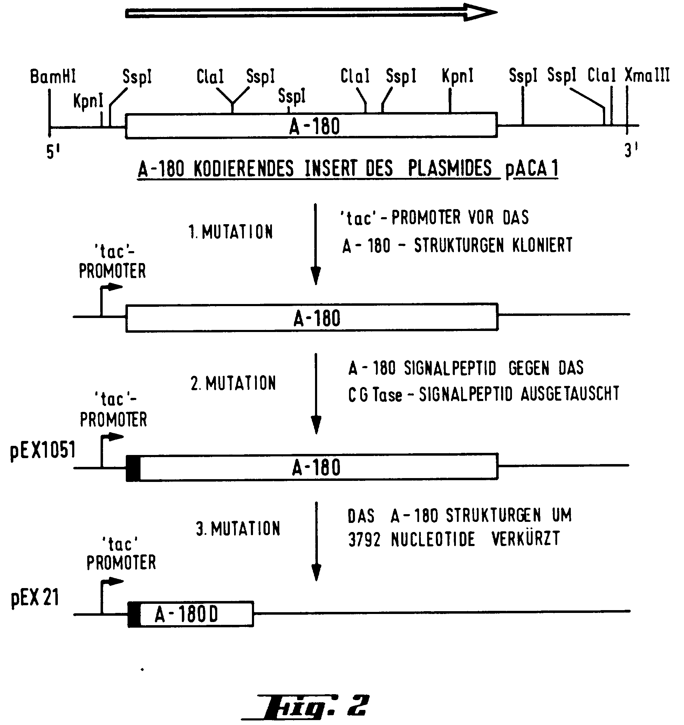

- Um einen Export der produzierten Amylase A-180 in den Kulturüberstand zu erreichen, wurden die 37 N-terminalen Aminosäuren von A-180, die das für den Export notwendige Signalpeptid darstellen, entfernt und durch das Signalpeptid der in E.coli exportierten CGTase aus Klebsiella oxytoca (Gene 47, 269-277,(1986)) ersetzt. Das rekombinante Plasmid wird als pEX1051 bezeichnet (Fig. 2). Die Expression des rekombinierten Gens erfolgte weiter über den 'tac'-Promotor.
Der Austausch des Signalpeptids ergab keinerlei Änderung im Exportverhalten von A-180. Das massiv produzierte Enzym liegt weiterhin cytoplasmatisch vor und wird stark abgebaut. Die G5 - Spezifität bleibt trotz des Signalpeptid- Austausches erhalten.

-Die dritte Mutation bestand darin, das A-180 - Strukturgen am 3'-Ende um 3792 Nukleotide zu verkürzen. Durch die Entfernung dieser Nukleotide und die Integration eines Stop-triplets an ihrer Stelle wird die Amylase am C-terminus um genau 1264 Aminosäuren verkürzt (Plasmid pEX21 (Fig.2)). Der verbliebene Amylaserest wird wie das gesamte A-180-Strukturgen nach Lactoseinduktion vom 'tac'-Promotor aus massiv expremiert. Im Gegensatz zur mutierten Gesamtamylase wird das gebildete Produkt nun jedoch in das Periplasma oder den Kulturüberstand von geeigneten E. coli-Stämmen exportiert. Das exportierte Protein ist stabil, d.h. es wird nicht abgebaut und seine enzymatischen Eigenschaften sind bezüglich der Produktspezifität identisch mit denen der kompletten Amylase A-180.

Dieses Genprodukt erfüllt damit alle Anforderungen, die zur G5-Produktion nötig sind.

### Beispiel 6: Expression und Sekretion der Amylase A-180 und des A-180 Derivates (A-180D) in verschiedenen E. coli Stämmen

Zur Expression der Amylase A-180 oder des G5-spezifischen 63 kDa - A-180 - Derivates A-180D werden die E. coli-Stämme HB101 und WCM100 verwendet. HB101 ist bei der Deutschen Sammlung von Mikroorganismen (DSM 1007) hinterlegt, WCM100 ist nach dem in EP-A-338 410 beschriebenen Verfahren erhältlich. Er kann für die Expression und Sekretion der Amylasen durch andere nach dem in EP-A-338410 offenbarten Verfahren erhältliche Stämme ersetzt werden. Die E. coli Stämme enthalten für die Expression von A-180 das Expressionsplasmid pEX 1051, für die Expression von A-180D das Expressions-, Sekretionsplasmid pEX21. 1000 ml Nährmedium (10 g/l Pepton aus Casein, 5 g/l Hefeextrakt, 10 g/l NaCl, 5 g/l Lactose und 0,1 g/l Ampicillin) werden mit 20 ml einer Vorkultur des jeweiligen Stammes (im gleichen Medium) inokuliert und aerob bei 20° C (pEX1051) bzw. 25° C (pEX21) inkubiert. Nach 48 h (pEX1051) bzw. 24 h (pEX21) werden die Zellen durch Zentrifugation der Kulturbrühe geerntet.

Bei Verwendung der Stämme HB101/pEX1051 und WCM100/pEX1051 werden die geernteten Zellen mit TC-Puffer gewaschen, in 1/200 Anzuchtvolumen TC-Puffer suspendiert und mit Hilfe von Ultraschall (Sonifier) oder Druck ('french press') lysiert. Die erhaltenen Zell-Lysate werden nach DNase-Behandlung 10 min bei 10000 x g zentrifugiert. Die Überstände nach dieser Zentrifugation, der im folgenden als Cytoplasmafraktion bezeichnet wird, enthalten die Amylase A-180 und können direkt zur Stärkekonversion verwendet werden.

Bei Verwendung des Stammes HB101/pEX21 wird die im Periplasma lokalisierte Amylase A-180D durch eine CHCl₃-Behandlung (Ames et al. (1984) J. Bact., 160;1181-1183) aus den Zellen extrahiert. Dazu werden die abzentrifugierten Zellen in 5 ml 10 mM Tris/HCl pH 8,0 aufgeschlämmt, mit 5 ml CHCl₃ vermischt und 15 min bei Raumtemperatur inkubiert. Die Suspension wird dann mit 40 ml TC-Puffer verdünnt und 20 min bei 6000 xg zentrifugiert. Nach der Zentrifugation wird das Zellpellet verworfen. Der Überstand (Periplasmafraktion) enthält 60-70% der gebildeten Amylase A-180D. Andere in der Periplasmafraktion enthaltene Proteine wirken nicht inhibierend auf die A-180D-Aktivität, so daß auf eine weitere Aufreinigung verzichtet werden kann.

Bei Verwendung des Stammes WCM100/pEX21 werden die geernteten Zellen verworfen. Der Kulturüberstand enthält unter den dargestellten Bedingungen 0,1 - 0,5 g des rekombinanten Genprodukts A-180D, während der Induktor Lactose zu diesem Zeitpunkt fast vollständig verbraucht ist. Der zellfreie Kulturüberstand kann direkt zur Stärkekonversion verwendet werden.

### Beispiel 7: Stärkekonversion mit Maltopentaose produzierenden Amylasen, die aus Isolat 163-26 oder E. coli gewonnen wurden.

### Beispiel 7.1: Stärkekonversion mit Amylase A-180, gereinigt aus dem Kulturüberstand von Isolat 163-26

Gereinigte Amylase A-180 wird in einer Konzentration von 50µg/ml in TC-Puffer gelöst. Eine 10 %-ige Lösung von löslicher Stärke in TC-Puffer wird auf eine Temperatur von 45°C gebracht. Die beiden Lösungen werden im Volumenverhältnis 1:1 gemischt und bei 45°C inkubiert. Nach 1 h wird die Reaktion durch die Zugabe von 1,5 Volumenteilen Methanol gestoppt. Die durch die Methanolzugabe ausgefällte unhydrolysierte Reststärke wird abzentrifugiert. Die in Lösung bleibenden Hydrolyseprodukte können durch eine 'reversed phase'- Säulenchromatografie qualitativ und quantitativ untersucht werden.
Bei einer typischen Stärkekonversion, bei der 1 ml Enzymlösung und 1 ml Substratlösung eingesetzt worden sind nach 1 h 18,5 % der im Ansatz enthaltenen Stärke hydrolysiert. Die daraus entstandenen Produkte weisen folgende Zusammensetzung auf: G5, 82,7 %; G4, 6,4 %; G3, 4,2 %; G2, 3,9 %; G1, 2,8 %.

### Beispiel 7.2: Stärkekonversion mit Amylase A-180, enthalten in den cytoplasmatischen Proteinfraktionen von E. coli - Zellen

Die cytoplasmatischen Proteinfraktionen von E. coli HB101/pEX1051 bzw E. coli WCM100/pEX1051 werden, wie in Beispiel 6 beschrieben, präpariert. Die Konzentration der Proteine wird mit TC-Puffer auf 2 mg/ml eingestellt. 35 ml einer 30 %-igen Noredux 150B - Lösung (in TC-Puffer) werden bei 45 °C äquilibriert. Noredux 150 B ist eine durch Säurebehandlung teilhydrolysierte Stärke der Firma Henkel. Das Substrat wird dann mit 5 ml der Proteinlösung (2 mg/ml) versetzt und bei 45 °C inkubiert. Nach 1,2,3 und 4 h werden jeweils 4 ml des Ansatzes entnommen, mit 6 ml Methanol versetzt und zentrifugiert. Die qualitative und quantitative Zusammensetzung der löslichen Produkte im jeweiligen Überstand wird durch HPLC - Analyse ermittelt.
Die Ergebnisse einer typischen Stärkekonversion mit Amylase A-180, enthalten in der cytoplasmatischen Proteinfraktion von E. coli HB101/pEX1051 bzw E. coli WCM100/pEX1051, sind in der folgenden Tabelle dargestellt:

| | 1 h | 2 h | 3 h | 4 h |
|---|---|---|---|---|
| | | | | |
| hydrolys. Substratanteil: | 12,1 % | 19,9 % | 24,95 % | 31,1 % |
| | | | | |
| Produktzusammensetzung: | | | | |
| Maltopentaose: | 100 % | 79 % | 72 % | 64 % |
| Maltotetraose: | 0 % | 8,5 % | 10,4 % | 12,2 % |
| Maltotriose: | 0 % | 7,5 % | 10,4 % | 11,6 % |
| Maltose: | 0 % | 5 % | 6,2 % | 7,4 % |
| Glucose: | 0 % | 0 % | 1,0 % | 4,8 % |

### Beispiel 7.3: Stärkekonversion mit Amylase A-180D, enthalten in der Periplasmafraktion von E. coli HB101/pEX21

95 ml 10%-ige Noredux 150B-Lösung werden bei 45°C äquilibriert. Dann wird die Lösung mit 5 ml Periplasmafraktion von E. coli HB101/pEX21 (vgl. Beispiel 6) vermischt und bei 45° inkubiert. Nach 1 h wird die Reaktion durch Zugabe von 150 ml Methanol gestoppt. Der Ansatz wird zentrifugiert; anschließend wird die Produktzusammensetzung im Überstand durch HPLC-Analyse bestimmt. Nach 1 h sind unter den beschriebenen Bedingungen 38,4% des eingesetzten Substrates hydrolysiert. Die entstandenen Produkte setzen sich wie folgt zusammen: G5, 67,7%; G4, 11,1%; G3, 1,7%; G2, 8,7%; G1 10,8%.

### Beispiel 7.4: Stärkekonversion mit Amylase A-180D, enthalten im Kulturüberstand von E. coli WCM100/pEX21

75 ml 10 %-ige Noredux 150 B - Lösung werden bei 45°C äquilibriert. Dann wird die Lösung mit 25 ml Kulturüberstand von E. coli WCM100/pEX21 (vgl. Beispiel 6) vermischt und bei 45 °C inkubiert. Nach 1 h wird die Reaktion durch Zugabe von 150 ml Methanol gestoppt. Der Ansatz wird zentrifugiert; anschließend wird die Produktzusammensetzung im Überstand durch HPLC-Analyse bestimmt.

Nach einer Stunde waren 15,8 % des eingesetzten Substrates verwertet. Die Produktzusammensetzung war:

| | |
|---|---|
| Maltopentaose | 91,3 % |
| Maltotetraose | 5,4 % |
| Maltotriose | 1,2 % |
| Maltose | 0,9 % |
| Glucose | 0.9 % |

## Patentansprüche (Patentansprüche für folgende(n) Vertragsstaat(en): BE, DE, FR, GB, IT, SE)

1. Maltopentaose produzierende Amylase, dadurch gekennzeichnet, daß sie die in Beispiel 4 wiedergegebene 1684 Aminosäure umfassende Sequenz besitzt.

2. Verfahren zur Herstellung von Amylasen, die Maltopentaose als einzige Hauptkomponente produzieren, dadurch gekennzeichnet, daß die komplette Nukleotidsequenz des Beispiels 4, die das dort angegebene Amylase Strukturgen umfaßt, für eine verstärkte Expression und/oder Sekretion modifiziert und in geeigneten Prokaryonten exprimiert bzw. exprimiert und sekretiert wird.

3. Verfahren zur Herstellung Maltopentaose produzierender Amylasen nach Anspruch 2, dadurch gekennzeichnet, daß das für eine Amylase nach Anspruch 1 kodierende Gen für eine verstärkte, steuerbare Expression in einem Vektor unter die Kontrolle eines induzierbaren Promotors gestellt wird.

4. Verfahren zur Herstellung Maltopentaose produzierender Amylasen nach einem der Ansprüche 2 oder 3, dadurch gekennzeichnet, daß bei dem für eine Amylase nach Anspruch 1 kodierende Gen die das Leaderpeptid kodierende DNA Sequenz unter Erhaltung des Leserasters gegen die das Leaderpeptid eines sekretierten Proteins kodierende DNA Sequenz ausgetauscht wird.

5. Verfahren zur Herstellung Maltopentaose produzierender Amylasen nach Anspruch 4, dadurch gekennzeichnet, daß als kodierende DNA-Sequenz für das Leaderpeptid eines sekretierten Proteins die DNA-Sequenz verwendet wird, die für das Leaderpeptid der CGTase von Klebsiella oxytoca kodiert.

6. Verfahren zur Herstellung Maltopentaose produzierender Amylasen nach Anspruch 5, dadurch gekennzeichnet, daß die ersten 111 kodierenden Basen des Strukturgens für die Amylase nach Anspruch 1 durch die DNA-Sequenz ersetzt werden, die für das Signalpeptid der CGTase aus Klebsiella oxytoca kodiert.

7. Verfahren zur Herstellung Maltopentaose produzierender Amylasen nach einem oder mehreren der Ansprüche 2 bis 6 dadurch gekennzeichnet, daß bei dem für eine Amylase nach Anspruch 1 kodierende Gen der 3'- Bereich verkürzt und mindestens ein Stopcodon am Ende des verkürzten Bereichs integriert wird.

8. Verfahren zur Herstellung Maltopentaose produzierender Amylasen nach Anspruch 7, dadurch gekennzeichnet, daß der 3'- Bereich des Amylase kodierenden Gens um 3792 Nukleotide verkürzt und mindestens ein Stopcodon am Ende des verkürzten Bereichs integriert wird.

9. Verfahren zur Herstellung Maltopentaose produzierender Amylasen nach einem oder mehreren der Ansprüche 2 bis 8 dadurch gekennzeichnet, daß als Produzent E. coli verwendet wird.

10. DNA Konstrukt kodierend eine Maltopentaose produzierende Amylase, dadurch gekennzeichnet, daß sie dadurch erhältlich ist, daß die komplette Nukleotidsequenz des Beispiels 4, die das dort angegebene Amylase-Strukturgen umfaßt, für eine verstärkte Expression und/oder Sekretion modifiziert wird.

11. Alkalophiles Isolat 163-26 (DSM 5853).

## Claims (Claims for the following Contracting State(s): BE, DE, FR, GB, IT, SE)

1. Maltopentaose producing amylase, characterized in that it has the sequence comprising 1684 amino acid [sic] reproduced in Example 4.

2. Process for preparing amylases which produce maltopentaose as single main component, characterized in that the complete nucleotide sequence of Example 4, which comprises the amylase structural gene indicated therein, is modified for enhanced expression and/or secretion and is expressed or expressed and secreted in suitable prokaryotes.

3. Process for preparing maltopentaose producing amylases according to Claim 2, characterized in that the gene coding for an amylase according to Claim 1 is placed under the control of an inducible promoter, for enhanced, controllable expression in a vector.

4. Process for preparing maltopentaose producing amylases according to one of Claims 2 or 3, characterized in that the DNA sequence encoding the leader peptide in the gene coding for an amylase according to Claim 1 is replaced, with retention of the reading frame, by the DNA sequence encoding the leader peptide of a secreted protein.

5. Process for preparing maltopentaose producing amylases according to Claim 4, characterized in that the DNA sequence which codes for the leader peptide of the CGTase of Klebsiella oxytoca is used as coding DNA sequence for the leader peptide of a secreted protein.

6. Process for preparing maltopentaose producing amylases according to Claim 5, characterized in that the first 111 coding bases of the structural gene for the amylase according to Claim 1 are replaced by the DNA sequence which codes for the signal peptide of the CGTase from Klebsiella oxytoca.

7. Process for preparing maltopentaose producing amylases according to one or more of Claims 2 to 6, characterized in that the 3' region of the gene coding for an amylase according to Claim 1 is truncated, and at least one stop codon is integrated at the end of the truncated region.

8. Process for preparing maltopentaose producing amylases according to Claim 7, characterized in that the 3' region of the amylase encoding gene is truncated by 3792 nucleotides, and at least one stop codon is integrated at the end of the truncated region.

9. Process for preparing maltopentaose producing amylases according to one or more of Claims 2 to 8, characterized in that E. coli is used as producer.

10. DNA construct encoding a maltopentaose producing amylase, characterized in that it is obtainable by modifying the complete nucleotide sequence of Example 4, which comprises the amylase structural gene indicated therein, for enhanced expression and/or secretion.

11. Alkalophilic isolate 163-26 (DSM 5853).

## Revendications (Revendications pour l'(les) Etat(s) contractant(s) suivant(s): BE, DE, FR, GB, IT, SE)

1. Amylase produisant de la maltopentose, caractérisée en ce qu'elle possède la séquence développée de 1684 acides aminés représentée à l'exemple 4.

2. Procédé de préparation d'amylases qui produisent de la maltopentose comme composant principal unique, caractérisé en ce que la séquence nucléotidique complète de l'exemple 4, qui comprend le gène de structure de l'amylase citée, est modifiée pour une expression et/ou sécrétion renforcées et est exprimée et respectivement, exprimée et secrétée, dans des procaryotes appropriés.

3. Procédé de préparation d'amylases qui produisent de la maltopentose suivant la revendication 2, caractérisé en ce que le gène codant pour une amylase suivant la revendication 1, est placé dans un vecteur sous le contrôle d'un promoteur inductible, pour une expression renforcée, réglable.

4. Procédé de préparation d'amylases qui produisent de la maltopentose suivant la revendication 2 ou 3, caractérisé en ce que dans le gène codant pour une amylase suivant la revendication 1, la séquence d'ADN codant la séquence de tête est échangée, avec maintien du cadre de lecture, avec la séquence d'ADN codant la séquence de tête d'une protéine secrétée.

5. Procédé de préparation d'amylases qui produisent de la maltopentose suivant la revendication 4, caractérisé en ce que l'on utilise comme séquence d'ADN codant pour la séquence de tête d'une protéine secrétée, la séquence d'ADN qui code pour la séquence de tête de la CGTase de Klebsiella oxytoca.

6. Procédé de préparation d'amylases qui produisent de la maltopentose suivant la revendication 5, caractérisé en ce que les 111 premières bases du gène de structure codant pour l'amylase suivant la revendication 1, sont remplacées par la séquence d'ADN qui code pour le peptide signal de la CGTase de Klebsiella oxytoca.

7. Procédé de préparation d'amylases qui produisent de la maltopentose suivant l'une ou plusieurs des revendications 2 à 6, caractérisé en ce que dans le gène codant pour une amylase suivant la revendication 1, le domaine 3' est raccourci et au moins un codon d'arrêt est intégré à l'extrémité du domaine raccourci.

8. Procédé de préparation d'amylases qui produisent de la maltopentose suivant la revendication 7, caractérisé en ce que le domaine 3' du gène codant l'amylase est raccourci de 3792 nucléotides et au moins un codon d'arrêt est intégré à l'extrémité du domaine raccourci.

9. Procédé de préparation d'amylases qui produisent de la maltopentose suivant l'une ou plusieurs des revendications 2 à 8, caractérisé en ce que E. coli est utilisé comme producteur.

10. Construction d'ADN codant pour une amylase produisant de la maltopentose, caractérisée en ce qu'elle peut être préparée par modification de la séquence complète en nucléotides de l'exemple 4, qui comprend le gène de structure de l'amylase citée, pour une expression et/ou une sécrétion renforcées.

11. Isolat alcalophile 163-26 (DSM 5853).
